# EUROPÄISCHE PATENTSCHRIFT

(11) **EP 2 488 127 B1**
(45) Veröffentlichungstag und Bekanntmachung des Hinweises auf die Patenterteilung: **23.07.2014**
(21) Anmeldenummer: 10784670.1
(22) Anmeldetag: 07.10.2010
(51) Int. Cl.: A61F 2/30, A61F 2/40

(54) **MODULARES SYSTEM ZUR VERANKERUNG UND POSITIONIERUNG VON KOMPONENTEN VON IMPLANTATEN**
MODULAR SYSTEM FOR ANCHORING AND POSITIONING COMPONENTS OF IMPLANTS
SYSTÈME MODULAIRE POUR LE POSITIONNEMENT ET L'ANCRAGE D'ÉLÉMENTS D'IMPLANTS

(30) Priorität: 12.10.2009 DE 102009049660
(43) Veröffentlichungstag der Anmeldung: 22.08.2012
(73) Patentinhaber: AAP Implantate AG, 12099 Berlin (DE)
(72) Erfinder: FISCHER, Hans-Joachim, 12277 Berlin (DE); SCHAUWECKER, Heinz, Helge, 14476 Potsdam (DE); STAHL, Jens-Peter, 44229 Dortmund (DE); KUNZ, Jörn, 61118 Bad Vilbel (DE)
(74) Vertreter: Meissner, Peter E.
(86) Internationale Anmeldenummer: PCT/DE2010/001190
(87) Internationale Veröffentlichungsnummer: WO 2011/044879

(56) Entgegenhaltungen:
- EP-A1- 0 581 667
- EP-A1- 1 787 603
- DE-U1-202005 013 075
- FR-A1- 2 846 550

## Beschreibung

Die Erfindung betrifft ein modulares System zur Verankerung und Positionierung von Komponenten von Implantaten, wie zum Beispiel der Glenoidkomponente einer Schulterprothese, der Femurkopfkomponente einer Hüftendoprothese (Teilprothese / Oberflächenersatz), einer Tibiakomponente eines Kniegelenkes.

Beim normalen (natürlichen) anatomischen Gelenke bewegt sich meistens eine konkave Gelenkfläche gegen eine konvexe Gelenkfläche.

Wird das natürliche Gelenk ersetzt durch eine Gelenkprothese, also zum Beispiel durch eine Schultergelenkprothese, so spricht man von einem Teil- oder Totalgelenkersatz, je nachdem ob nur eine oder beide Gelenkflächen ersetzt werden.

Hierbei stellt die dauerhafte und biomechanisch vertretbare Positionierung und Verankerung der Implantatkomponenten, speziell bei der Verankerung von Komponenten im Glenoid, der proximalen Tibia und dem Oberflächenersatz am Femurkopf bzw. den Kniekondylen häufig ein Problem dar.

In der zu verankernden Gelenkkomponente ist ein (zentrale) Befestigungsschraube vorgesehen und üblicherweise kommen zusätzliche Verankerungen in Form von versetzt zueinander angeordneten (winkelstabilen) Schrauben zum Einsatz, die die Gelenkkomponente mit dem korrespondierenden Gelenkbereich (Knochen) zusätzlich verbinden bzw. in diesem halten.

Hierbei können eine Reihe von Problemen auftreten, und zwar insbesondere hinsichtlich des dauerhaften Haltes der Schrauben in dem korrespondierenden Gelenkbereich wegen des teilweise nur sehr begrenzten oder osteoporotischen Knochenmaterials, das im Verbindungsbereich vorhanden ist.

Hinzu kommt, dass durch die Gelenkbewegungen und Gelenkbelastungen diese Verankerungen einer stetigen zum Teil sehr unphysiologischen Krafteinwirkung unterworfen sind.

Der nächstliegende Stand der Technik wird in EP-A-1 787 603 offenbart.

Der Erfindung liegt die Aufgabe zugrunde, eine Verankerungsmöglichkeit für die Gelenkkomponenten bereitzustellen, die eine stabile, dauerhafte und physiologische Positionierung, Fixierung und Krafteinleitung gewährleistet.

Gelöst wird diese Aufgabe erfindungsgemäß durch eine Glenoidprothese, umfassend
- ein an die Gelenkanatomie und Knochenbeschaffenheit angepasstes, eine Implantatkomponente bildendes Formelement (1), dass durch ein vorzugweise zentrales Befestigungselement (3) winkelstabil mit dem Knochen verbindbar ist,
- wobei das Befestigungselement auch der Anbindung weiterer Implantatkomponenten dient,
- und durch in das Formelement und den Knochen divergierend oder konvergierend einbringbare, klingenartige Fixierelemente (5), die sich mit ihrem hinteren Ende am Formelement (1) abstützen.

Diese winkelstabile Fixierung der Formelemente erfolgt somit mittels des Befestigungselementes und oder mehrerer Schrauben die zusätzlich auch zur Positionierung und Befestigung der Implantatkomponenten mit herangezogen werden können

Der besondere Vorteil der erfindungsgemäßen Lösung ist darin zu sehen, dass durch die speziell auf jeweiligen Gelenkanatomien und Knochenbeschaffenheiten auslegbaren und in ihrer Geometrie und Größe anpassbaren und beliebig kombinierbaren Fixierelemente die Komponenten der Implantate winkelstabil fixiert werden können. Erfindungsgemäß sind die Fixierelemente flach und klingenartig ausgeführt um eine, besonders bei divergierender oder konvergierender Positionierung, größtmögliche projizierte Fläche zur optimalen Krafteinleitung zu erreichen.

Diese Fixierelemente sind, entsprechende den anatomischen und biomechanischen Gegebenheiten in einem dadurch vorgegeben Winkel zur der Längsachse des Befestigungselementes konvergierend oder divergierend ausgerichtet und weisen vorzugsweise einen abgewinkelten Kontaktbereich mit der zu verankernden Gelenkkomponente auf, der sich nach dem Einbringen der Formelemente auf der Implantatkomponente abstützten kann.

Es ist vorteilhaft, wenn dazu eine dem Kontaktbereich entsprechende Ausnehmung in der Implantatkomponente vorgesehen ist.

Um die Stabilität zu verbessern, können die Fixierelemente zusätzlich eine Längsprofilierung aufweisen, wobei die Längsprofilierung eine Längsführung für einen gesonderten, temporär einsetzbaren Positionierungsstift aufweisen kann.

Die Erfindung soll nachfolgend mit Bezug auf die Zeichnungen näher erläutert werden.

Dabei zeigt:
- Fig. 1: schematisch beispielhaft eine Implantatkomponente für das Schultergelenk mit einer Glenoidkomponente und dem Schultergelenkkopf,
- Fig. 2 und Fig. 3: Detaildarstellungen der klingenartigen Fixierelemente

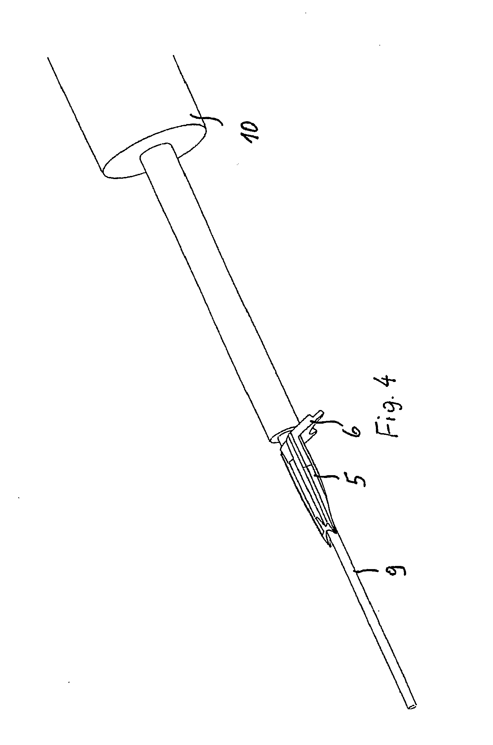

- Fig. 4: eine schematische Darstellung eines Positionierwerkzeuges.

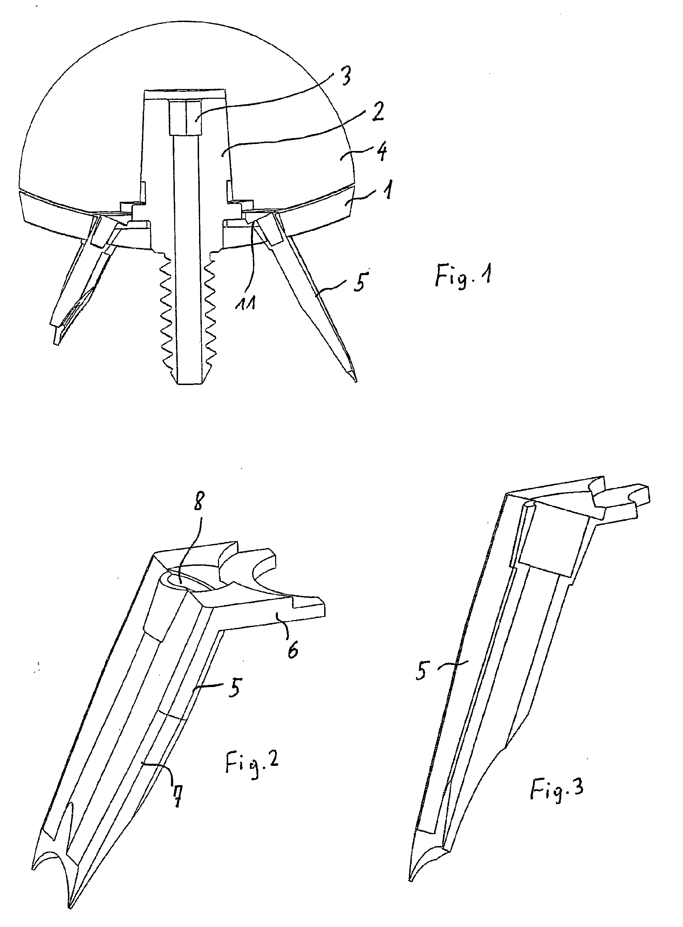

Die Figur 1 zeigt schematisch einen Gelenkkugelkopf 4, wie er für eine Schultergelenkprothese verwendet werden kann.

Das mit 1 bezeichnete Formelement oder die Implantatkomponente wird mit dem Glenoid mittels des Befestigungselementes 3 winkelstabil verbunden. Diese Verankerung wird durch zusätzliche klingenartige Fixierelemente 5 gesichert. Auf den Konus 2 ist der Gelenkkugelkopf 4 aufgesetzt.

Wie aus der Figur 1 ersichtlich, sind die klingenartigen Fixierelemente 5 gegenüber der Längsachse des Befestigungselementes 3 geneigt, und zwar in einem anatomisch und biomechanisch angepassten Winkel (divergierend oder konvergierend) zur Längsachse des Befestigungselementes 3.

Die Figuren 2 und 3 zeigen die Ausbildung der klingenartigen Fixierelemente 5 im Detail, wobei erkennbar ist, dass diese eine Längsprofilierung aufweisen, die mit 7 bezeichnet ist. Außerdem ist eine Abwinkelung 6 vorgesehen, die den Kontaktbereich zu dem zu fixierenden Formelement darstellt. Nach dem Einbringen liegt dieser Kontaktbereich vorzugsweise in einer korrespondierenden Ausnehmung 11 in dem Formelement 1.

In der Figur 2 ist noch eine Führung 8 erkennbar.

Beim Einsetzen der Implantatkomponente z.B. in das Glenoid kann zunächst ein Positionierungsdraht oder-stift 9 in das Knochenmaterial getrieben werden. Über diesen wird dann das klingenartige Fixierelement 5 aufgeschoben und mittels eines externen Werkzeuges 10 positionsgenau durch die Implantatskomponente in den Knochen eingetrieben.

## Patentansprüche

1. Glenoidprothese, umfassend
- ein an die Gelenkanatomie und Knochenbeschaffenheit angepasstes, eine Implantatkomponente bildendes Formelement (1), dass durch ein zentrales Befestigungselement (3) winkelstabil mit dem Knochen verbindbar ist,
- wobei das Befestigungselement auch der Anbindung einer Kugelkomponente (4) dient,
- und wobei in das Formelement und den Knochen ein oder mehrere zur Längsachse des Befestigungselementes (3) divergierend oder konvergierend einbringbare Fixierelemente (5) vorgesehen sind, die sich mit ihrem hinteren Ende am Formelement (1) abstützen,
**dadurch gekennzeichnet,**
**dass** die Fixierelemente (5) klingenartig ausgebildet sind.

2. Glenoidprothese nach Anspruch 1,
**dadurch gekennzeichnet,**
**dass** die Fixierelemente (5) einen abgewinkelten Kopfbereich (6) aufweisen, der sich nach dem Einbringen auf dem Formelement (1) abstützt.

3. Glenoidprothese nach einem der vorstehenden Ansprüche,
**dadurch gekennzeichnet,**
**dass** die Fixierelemente (5) jeweils eine oder mehrere Schneiden aufweisen.

4. Glenoidprothese nach einem der vorstehenden Ansprüche,
**dadurch gekennzeichnet,**
**dass** die Schneiden eine Längsprofilierung (7) aufweisen.

5. Glenoidprothese nach einem der vorstehenden Ansprüche,
**dadurch gekennzeichnet,**
**dass** die Längsprofilierung (7) eine Längsführung (8) für einen gesonderten Positionierungsstift (9) aufweist.

## Claims

1. A glenoid prosthesis comprising
- a shaped element (1), adapted to the joint anatomy and bone condition and forming an implant component, which is connectable to the bone by a central securing element (3) in an angularly stable manner,
- wherein the securing element also serves to fasten a ball component (4),
- and wherein there are provided one or a plurality of fixing elements (5) insertable into the shaped element and the bone in a manner so as to be diverging or converging in relation to the longitudinal axis of the securing element (3), the respective rear ends of which fixing elements (5) are supported on the shaped element (1),
**characterised in that** the fixing elements (5) are blade-like.

2. A glenoid prosthesis according to claim 1, **characterised in that** the fixing elements (5) have an angled head region (6) which is supported on the shaped element (1) after insertion.

3. A glenoid prosthesis according to any one of the preceding claims, **characterised in that** the fixing elements (5) each have one or a plurality of cutting edges.

4. A glenoid prosthesis according to any one of the preceding claims, **characterised in that** the cutting edges have longitudinal profiling (7).

5. A glenoid prosthesis according to any one of the preceding claims, **characterised in that** the longitudinal profiling (7) has a longitudinal guide (8) for a separate positioning pin (9).

## Revendications

1. Prothèse glénoïde, comprenant
- un élément conformé (1) formant un composant d'implant adapté à l'anatomie de l'articulation et à la structure osseuse, qui peut être relié à l'os de manière à obtenir une stabilité angulaire par le biais d'un élément central de fixation (3),
- l'élément de fixation servant également à la liaison d'un composant sphérique (4),
- et un ou plusieurs éléments de fixation (5) qui peuvent être introduits de manière divergente ou convergente par rapport à l'axe longitudinal de l'élément de fixation (3) dans l'élément conformé et l'os étant prévus, qui s'appuient au niveau de l'élément conformé (1) avec leur extrémité arrière,
**caractérisée**
**en ce que** les éléments de fixation (5) sont configurés en forme de lames.

2. Prothèse glénoïde selon la revendication 1,
**caractérisée**
**en ce que** les éléments de fixation (5) présentent une zone de tête courbée (6), qui s'appuie sur l'élément conformé (1) après l'introduction.

3. Prothèse glénoïde selon l'une des revendications précédentes,
**caractérisée**
**en ce que** les éléments de fixation (5) présentent respectivement une ou plusieurs arêtes.

4. Prothèse glénoïde selon l'une des revendications précédentes,
**caractérisée**
**en ce que** les arêtes présentent un profilage longitudinal (7).

5. Prothèse glénoïde selon l'une des revendications précédentes,
**caractérisée**
**en ce que** le profilage longitudinal (7) présente un guidage longitudinal (8) pour une broche de positionnement séparée (9).
